# EUROPEAN PATENT APPLICATION

(11) **EP 0 716 861 A1**
(43) Date of publication of application: **19.06.1996**
(21) Application number: 95308107.2
(22) Date of filing: 13.11.1995
(51) Int. Cl.: A61M 16/18

(54) **Pump flow vaporiser**

(30) Priority: 21.11.1994 US 342549
(71) Applicant: OHMEDA INC., Liberty Corner, New Jersey 07938-0804 (US)
(72) Inventor: Mashak, James N., Sun Prairie, Wisconsin 53590 (US)
(74) Representative: Gough, Peter

(57) **Abstract**

A vaporiser for introducing a vapour of an anaesthetic liquid into a carrier gas for administration to a patient is disclosed. The vaporiser directly vaporises the liquid by introducing the liquid into a vaporising chamber having an extremely large width to height ratio, i.e. in excess of 600:1. The carrier gas flows in laminar flow through the vaporising chamber and the liquid anaesthetic is introduced at a controlled flow such that it immediately vaporises upon introduction to the entrance of the vaporising chamber. A control scheme utilising a CPU controls the percentage of anaesthetic vapour in the outlet gas by sensing certain parameters and then controlling the flow of liquid anaesthetic by controlling the speed of a controllable speed pump.

## Description

The present invention relates to an apparatus for vaporising a liquid and, more particularly, to a vaporiser that receives carrier gas and vaporises a liquid anaesthetic agent with that carrier gas to produce a stream of carrier gas containing a known concentration of anaesthetic agent for introduction into a patient.

There are, various methods and means of vaporising anaesthetic agents to provide an anaesthetising gas to a patient. Typical of one of the types, is that described in US patent 4,059,657 of Hay. More recently, with the introduction of an anaesthetic agent having a relatively low boiling point, newer methods of vaporising anaesthetic agents have been devised and one such method and apparatus is described in US 5,146,915 of Montgomery.

In the typical current anaesthetic vaporisers, the unit contains a sump where liquid anaesthetic is maintained and which also includes various wicks that provide a surface for enhancing the vaporisation of that anaesthetic. An inlet receives the carrier gas from a pressurised source, and generally, the stream of carrier gas is divided in the vaporiser such that a main stream of carrier gas continues toward the outlet while a bypass stream is diverted from the main stream and which enters the vaporising chamber and picks up anaesthetic vapour from the sump and wick arrangement. That bypass stream, now saturated with anaesthetic later recombines with the main stream of carrier gas to pass through the outlet to be administered to a patient.

Control of the amount of anaesthetic vapour in such vaporisers is accomplished by changing the amount of flow in the bypass flow path as a proportion of the carrier gas that passes through the vaporiser. As can be seen with reference to the cited prior art patents, the vaporisers, therefore, required a sump and which had to be at least of sufficient volume to contain enough liquid anaesthetic to be used in the operating room during an operation. In addition, the mass of the vaporisers was also somewhat large to maintain, to the extent possible, a constant temperature throughout the operation of the vaporiser.

Such vaporisers are limited to relatively low flows due to the need to saturate the bypass stream with liquid agent before recombining with the main stream of carrier gas and, as noted, the vaporisation occurred in a bypass stream, generally of a small proportion as opposed to the main stream. Thus the overall flow is limited and the vaporising chamber itself must be separate from the main path of the carrier gas passing through the vaporiser.

Accordingly, such present vaporisers are generally large, heavy in mass and are somewhat limited in their ability to provide high flows of an anaesthetic laden gas to a desired use.

It is an aim of the present invention to provide an anaesthetic vaporiser which overcomes the difficulties and problems heretofore associated with anaesthetic vaporisers.

According to the present invention an anaesthetic vaporiser comprises an inlet for receiving carrier gas and an outlet for carrier gas and vaporised anaesthetic agent for delivery to a patient, a vaporising chamber forming a flow path between said inlet and said outlet, said vaporising chamber having a large width to height ratio and forming a flow field for the flow of carrier gas therethrough, a liquid anaesthetic agent inlet in said vaporising chamber for admitting liquid anaesthetic agent at a predetermined location into the flow of carrier gas through said vaporising chamber, and a means of supplying liquid anaesthetic agent under predetermined pressure to said liquid anaesthetic agent inlet, said liquid being vaporised immediately upon entering said vaporising chamber to enter the flow of carrier gas passing through said vaporising chamber.

In particular, the present vaporiser provides direct vaporisation of the anaesthetic into the main flow of carrier gas and does not, therefore, include a bypass stream in order to create and deliver a suitable anaesthetising mixture to the patient. In the present invention, a vaporising chamber is of a specific shape, that is, having an extremely large width to height ratio and the carrier gas is introduced into that special vaporising chamber. The flow of carrier gas through the vaporising chamber is basically a laminar flow.

The liquid anaesthetic is introduced directly into that main laminar flow of carrier gas through one or more specially constructed openings and at those openings, vaporisation occurs immediately as the liquid reaches the vaporising chamber. Due to the particular configuration of the opening or openings for the liquid anaesthetic agent, a large gas/liquid interface area is formed inside the vaporising chamber at the entrance of the opening(s). It is important to note that the liquid anaesthetic does not enter the vaporising chamber to any real extent as the flow is controlled such that the liquid is vaporised immediately as it enters the vaporising chamber and only a meniscus is formed at the entrance to the vaporising chamber. Thus, the gas/liquid interface provides immediate vaporisation at the entrance to the vaporising chamber.

Again, due to the extremely small height of the vaporising chamber, the volume of carrier gas passing through the vaporising chamber instantly vaporises the liquid anaesthetic agent immediately upon entrance into the vaporising chamber. The liquid anaesthetic is therefore delivered directly to the vaporising chamber under a positive pressure, such as by a pump, and the supply of liquid anaesthetic may, therefore, be at some more remote location. Accordingly, the anaesthetic vaporiser itself does not contain, nor need, a sump for containing the liquid anaesthetic.

Control of the concentration of anaesthetic vapour in the outlet gas is easily achieved by controlling the main flow of the carrier gas and the flow of the liquid anaesthetic. That control may be carried out by the design of the number and size of openings through which the liquid anaesthetic passes to reach the vaporising chamber as well as by controlling the pressure of the liquid anaesthetic that is so introduced.

By means of the present vaporiser, therefore, high flows are attainable and the liquid is directly vaporised in a vaporising chamber, thereby eliminating the need for bypass streams, proportioning valves and the like within the vaporiser. Additionally, the supply of liquid need not be provided by a large sump as part of the vaporiser.

An embodiment of the invention will now be described, by way of example, reference being made to the Figures of the accompanying diagrammatic drawings in which:-
Figure 1 is a cross-sectional schematic view of an anaesthetic vaporiser constructed in accordance with the present invention; and
Figure 2 is a top schematic view of the vaporiser of Figure 1.

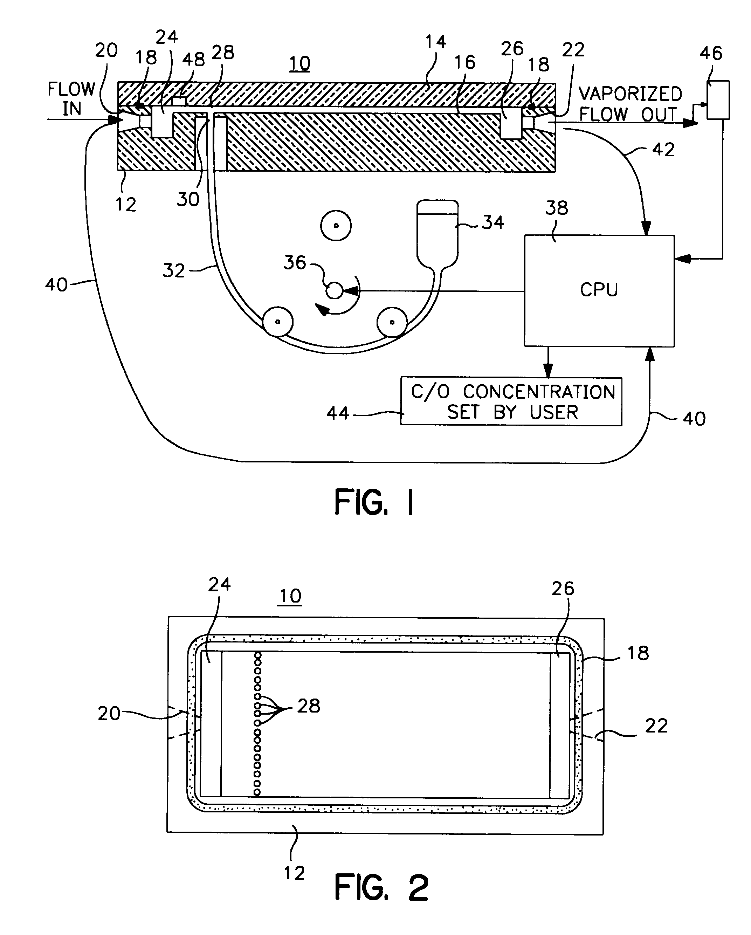

Referring now to Figures 1 and 2, there is shown an anaesthetic vaporiser assembly 10 comprising a base 12 and a cover 14, the latter of which may be of a transparent material such as a high strength polycarbonate to enable the user to view the interior of the anaesthetic vaporiser assembly 10 to monitor any build-up of material that could impede the performance of the anaesthetic vaporiser assembly 10.

Intermediate the cover 14 and the base 12, there is formed a vaporising chamber 16 which, in the preferred embodiment, is an elongate chamber, rectangularly shaped. The vaporising chamber 16 is encircled by a gasket 18 to seal its periphery between cover 14 and base 12. As an important feature of the present invention, the ratio between the length of the vaporising chamber 16 to its height is at least a predetermined value such that the vaporising chamber 16 is extremely narrow so as to promote laminar flow of the gas passing therethrough and to increase the boundary layer, that is, the contact with the walls of the vaporising chamber 16.

In the preferred embodiment, the vaporising chamber 16 is rectangular shaped, however, it may also be a circular or annular shape providing the width to height ratio is maintained at least at the predetermined value.

An inlet 20 is formed in the anaesthetic vaporiser assembly 10 which receives a carrier gas, such as oxygen. The carrier gas picks up anaesthetic vapour to produce a mixture of carrier gas/anaesthetic vapour for administration to a patient. An outlet 22 is formed at the opposite end of the vaporising chamber 16 from the inlet 20 and which channels the flow of anaesthetic laden carrier gas from the vaporiser assembly 10.

The vaporising chamber 16 is effectively a passage that communicates between the inlet 20 and the outlet 22 and as such receives carrier gas from the inlet 20 and delivers that gas to the outlet 22. To stabilise the flow of gas through the vaporiser assembly, there may also be an inlet plenum 24 and an outlet plenum 26.

Again, in the preferred embodiment, the width of the vaporising chamber 16 is about 3.0 inches and the height thereof is about .003 inches, which gives an overall width to height ratio of 1000:1. It is preferred that the width to height ratio be at least 600:1 to achieve the desired flow of gas through the vaporising chamber 16 and to enhance the vaporisation of liquid anaesthetic therein.

The liquid anaesthetic is preferably supplied to the vaporising chamber 16 by means of a plurality of holes 28 formed in the base 12 and which communicate between a manifold 30 to the interior of the vaporising chamber 16. As may be seen in Figure 2, in particular, in the preferred embodiment, the plurality of holes 28 are aligned in a row which is generally perpendicular to the flow of gas passing through the vaporising chamber 16. The uniform distribution of the holes 28 is important to insure that a uniform mixture of the vaporised liquid anaesthetic enters the stream of carrier gas. As such, extremely minute holes are preferred, typical being about .005 inches in diameter and spaced about .020 inches apart centreline to centreline along the row. As a alternative, an extremely narrow slit may be employed to introduce the liquid and may be in the order of .002 inches wide.

The size of the opening that introduces the liquid anaesthetic needs to be minute to obtain the desired distribution of liquid anaesthetic and to insure that the liquid is vaporised immediately upon introduction into the vaporising chamber 16.

A suitable conduit 32 delivers the liquid anaesthetic agent to the manifold 30 from a reservoir 34 that contains the supply of liquid anaesthetic agent. As may be seen, therefore, the reservoir 34 can readily be remote from the vaporising chamber 16 and thus easy to fill, clean etc. without disassembly of a vaporiser as is currently necessary in many vaporisers.

A pump 36 is located intermediate the reservoir 34 and the manifold 30 which is precisely controllable in speed so as to afford control of the flow of liquid anaesthetic agent being supplied to the manifold 30 and thus, to the holes 28 for introduction into the vaporising chamber 16. A typical pump 36 suitable for the purpose is a peristaltic pump which is readily controllable in speed to be sufficiently accurate for the use herein.

In use, the flow of carrier gas enters the inlet 20, and passes through the inlet plenum 24 where the flow is stabilised and distributed across the entrance to the vaporising chamber 16. The vaporising chamber 16 itself is generally rectangular in cross section and is extremely wide and thin, that is the width may be in the order of about 3.0 inches with its height being about .003 inches. Accordingly the overall width to height ratio is about 1000:1. It is important that such ratio be extremely high as will become apparent. typically a ratio in excess of 600 to 1. As such, the carrier gas entering into the vaporising chamber 16 is rapidly converted to a laminar flow that is generally uniformly spread across the width of the opening to the vaporising chamber 16.

The flow of carrier gas is therefore essentially laminar as it passes by the holes 28 through which the liquid anaesthetic is introduced into the vaporising chamber 16. The liquid anaesthetic, pressurised by the pump 36 enters the vaporising chamber 16 and is vaporised immediately. As indicated, at most, a meniscus of liquid is formed at the inlet to the vaporising chamber 16 since vaporisation is immediate as the liquid reaches vaporising chamber 16. The flow of carrier gas is maintained at a predetermined flow passing through the vaporising chamber 16 and the production of vapour is controlled by the flow of liquid anaesthetic agent by the pump 36.

The anaesthetic vapour is picked up by the carrier gas and is carried along the vaporising chamber 16 to emerge from the outlet 22 as a carrier gas containing a desired amount of anaesthetic vapour for administration to a patient. The amount of vapour produced, and thus the concentration of vapour in the stream of gas delivered to the patient is readily controlled by controlling the pump 36, as will be explained.

As a typical control scheme for the vaporiser, a CPU 38 may be used which gathers various data to control the pump 36. The CPU 38 gathers information via data line 40 indicating the pressure of carrier gas at the inlet 20 of the vaporising chamber 16 as well as via data line 42 indicating the pressure of carrier gas/vapour exiting the vaporising chamber 16 through the outlet 22. The measurement of pressures at data lines 40 and 42 represent the differential pressure across the vaporising chamber 16 and both may be conventional pressure sensors. Since the flow is laminar, that differential pressure provides an accurate determination of the flow through the vaporising chamber and which value can be used by the CPU 38 to determine the speed of the pump 36. CPU 38 also includes an input device, such as a keyboard 44 so that the user can set the desired concentration of anaesthetic laden gas to be delivered to the patient. That set concentration thus determines the speed of the pump 36 and can be empirically determined, knowing the flow of the carrier gas through the vaporising chamber 16, to set the proper speed of pump 36 to deliver the desired anaesthetic concentration. As a further feature, a heater 48 may be placed at the entrance to the vaporising chamber 16 to stabilise the temperature of the gas entering the vaporising chamber 16.

To supplement the control, an agent sensor 46 of a standard commercial type may be located to determine the actual concentration of anaesthetic in the gas to the patient and that value fed into the CPU 38. By a conventional feedback system, therefore, the agent sensor 46 may establish and control the desired concentration of anaesthetic in the gas delivered to the patient.

## Claims

1. An anaesthetic vaporiser 10 comprising an inlet 20 for receiving carrier gas and an outlet 22 for carrier gas and vaporised anaesthetic agent for delivery to a patient, characterised by a vaporising chamber 16 forming a flow path between said inlet 20 and said outlet 22, said vaporising chamber 16 having a large width to height ratio and forming a flow field for the flow of carrier gas therethrough, a liquid anaesthetic agent inlet 28 in said vaporising chamber 16 for admitting liquid anaesthetic agent at a predetermined location into the flow of carrier gas through said vaporising chamber 16, and a means 36 of supplying liquid anaesthetic agent under predetermined pressure to said liquid anaesthetic agent inlet 28, said liquid being vaporised immediately upon entering said vaporising chamber 16 to enter the flow of carrier gas passing through said vaporising chamber.

2. An anaesthetic vaporiser as claimed in Claim 1 wherein said means to supply liquid anaesthetic agent comprises a controllable speed pump 36.

3. An anaesthetic vaporiser as claimed in Claim 1 or 2 wherein said liquid inlet comprises a plurality of small openings 28 entering into said vaporising chamber 16.

4. An anaesthetic vaporiser as claimed in Claim 3 wherein said small openings are aligned along a path generally perpendicular to the flow of carrier gas passing through said vaporising chamber 16.

5. An anaesthetic vaporiser as claimed in claim 3 or 4 wherein said holes 28 are about .005 inches in diameter and the vaporising chamber 16 is rectangular in cross section.

6. An anaesthetic vaporiser as claimed in any one of Claims 1 to 5 wherein the width to height ratio is at least about 600:1.

7. An anaesthetic vaporiser as claimed in any one of Claims 1 to 5 wherein said width to height ratio is about 1000:1.

8. An anaesthetic vaporiser as claimed in any one of claims 1 to 7, in which a control means is provided to control the concentration of anaesthetic vapour in the flow of carrier gas and vaporised anaesthetic agent passing through said outlet 22 for delivery to a patient.

9. An anaesthetic vaporiser as claimed in any one of Claims 2 to 8 wherein said means to control the speed of said pump includes a CPU 38 and said pump speed is determined by an input to said CPU 38.
